# EUROPÄISCHE PATENTSCHRIFT

(11) **EP 0 740 532 B1**
(45) Veröffentlichungstag und Bekanntmachung des Hinweises auf die Patenterteilung: **22.07.1998**
(21) Anmeldenummer: 95905551.8
(22) Anmeldetag: 16.01.1995
(51) Int. Cl.: A61B 17/34

(54) **TROKARSYSTEM**
TROCAR SYSTEM
SYSTEME DE TROCART

(30) Priorität: 18.01.1994 DE 4401237
(43) Veröffentlichungstag der Anmeldung: 06.11.1996
(73) Patentinhaber: WILLY RÜSCH AG, D-71394 Kernen-Rommelshausen (DE)
(72) Erfinder: BUESS, Gerhard, D-72074 Tübingen (DE); MELZER, Andreas, D-65199 Wiesbaden (DE); JAKOUBEK, Franz, D-78576 Liptingen (DE); KRAUTER, Joachim, D-71404 Korb (DE)
(74) Vertreter: KOHLER SCHMID + PARTNER
(86) Internationale Anmeldenummer: DE9500058
(87) Internationale Veröffentlichungsnummer: WO9519146

(56) Entgegenhaltungen:
- EP-A- 0 474 124
- EP-A- 0 535 974
- DE-A- 4 134 655
- DE-A- 4 312 147
- DE-U- 9 112 550

## Beschreibung

Die Erfindung geht aus von einem Trokarsystem, das einen ein Lumen begrenzenden biegbaren Trokartubus, einen Trokarstössel und einen Ventilmechanismus aufweist, der am vom Patienten wegweisenden Ende des Trokartubus ausgebildet ist und durch dessen Ventilgehäuse hindurch der Trokarstössel in den Trokartubus einführbar ist.

Derartige Trokarsysteme sind aus der DE-OS 43 12 147 bekannt. Diese Trokarsysteme umfassen einen Obturator aus rostfreiem Stahl oder einem anderen steifen Material und eine Kanüle aus einem Kunststoff wie PTFG, Polyurethan oder PVC. Die aus einem derartigen Kunststoff gefertigte Kanüle weist ein proximales, offenes Ende und ein distales, offenes Ende auf und ist biegsam, um das Einführen eines bogenförmig gekrümmten chirurgischen Werkzeuges durch die Kanüle in die Körperhöhle zu ermöglichen, ohne daß deren Innendurchmesser vergrößert werden muß.

Aus der DE-OS 41 34 655 ist eine atraumatische Kanüle bekannt, bei der der Außenquerschnitt des Mandrins und der Innenquerschnitt des Schaftes im Bereich der Spitze des Schaftes mit einer Verjüngung versehen sind, wodurch beim Einbringen der Kanüle in das Gewebe der Druck auf den Mandrin bewirkt, daß dessen Verjüngung gegen die Verjüngung des Schaftes stößt und diesen "mitzieht" und sich dabei unter Vermeidung von Verwerfungen seines Materials streckt.

Das Trokarsystem gemäß der DE-OS 43 12 147 ist grundsätzlich für die Einführung eines gebogenen Operationsinstrumentes in einen Körperhohlraum geeignet. Über die Operationsinstrumente kann aber der Trokartubus im Gewebe auch möglicherweise hin- und herbewegt bzw. verschoben werden. Deshalb muß ein Trokartubus insbesondere bei der Einführung stark gebogener Instrumente, im Gewebe sicher verankert sein.

Der Erfindung liegt daher die Aufgabe zugrunde, bekannte flexible Trokarsysteme derart weiterzubilden, daß die Verankerung des Trokartubus im Gewebe verbessert ist und daß auch die Einführung von stärker gebogenen Instrumenten in den Trokartubus sicher möglich ist.

Diese Aufgabe wird erfindungsgemäß dadurch gelöst, daß der Trokartubus bezüglich der axialen Länge dehnbar ist, daß der Trokartubus am patientenseitigen Ende eine Verjüngung aufweist, die eine zirkuläre Stufe oder einen konusförmig verlaufenden Abschnitt an der Spitze des Trokarstössels formschlüssig umgreift.

Die Verjüngung ist derart ausgebildet, daß sie eng im Spitzenbereich des Trokarstössels formschlüssig anliegen kann. Dabei umgreift die Verjüngung eine zirkuläre Stufe oder einen konusförmig verlaufenden Abschnitt an der Spitze des Trokarstössels. Die formschlüssige Verbindung kann axial wirkende Kräfte, die entgegen der Plaziervorschubrichtung des Trokartubus wirken, aufnehmen. Am Patienten abgewandten Ende des Trokartubus ist ein Bund ausgeformt, der im Ventilgehäuse lagerbar ist. Der Bund kann über Einlagen im Bundmaterial oder über Stützringe stabilisiert werden und ist im Ventilgehäuse axial unverrückbar gehalten. In weiteren Ausführungsformen der Erfindung wäre es auch möglich, daß der Trokartubus im Ventilgehäuse drehbar gehalten ist.

Ein nach dieser technischen Lehre ausgebildeter Trokar hat den Vorteil, daß der Trokartubus definiert am Trokarstössel vorgespannt werden kann. Eine unzulässige Überdehnung des Trokartubus kann damit ausgeschlossen werden. Ist der Trokartubus vorgespannt, so kann er beispielsweise über das Ventilgehäuse mit dem Trokarstössel lösbar verbunden werden. Dabei stützt sich der Trokartubus einerseits an der zirkulären Stufe des Trokarstössels ab und andererseits wird der Trokartubus bevorzugt über sein maschinenseitiges Ende am Trokarstössel befestigt. Ist der Trokarstösselschaft dünner als seine Spitze, so kann der Trokarstössel leicht im Trokartubus gleiten, bis die Spitze des Trokarstössels formschlüssig in die Verjüngung des Trokartubus eingreift.

Ein weiterer Vorteil eines erfindungsgemäß ausgebildeten Trokars besteht darin, daß sich beim Längen des Trokartubus der Querschnitt des Trokartubus verkleinert. Nach dem Entfernen des Trokarstössels aus dem Trokartubus zieht sich dieser wieder zusammen und verkürzt sich somit. Dabei vergrößert sich der Querschnitt des Trokartubus. Diese Querschnittsvergrößerung bewirkt, daß der Trokartubus noch fester als zuvor im Gewebe verankert wird. Dadurch wird einerseits bewirkt, daß die traumatische Wirkung des Tubus im Gewebe verringert wird und daß andererseits die Befestigung bzw. Verankerung des Trokartubus im Gewebe deutlich erhöht wird, so daß das Verrutschen des Trokartubus während der Operation verhindert wird.

Ein weiterer Vorteil besteht darin, daß durch die bessere Verankerung des Trokartubus im Gewebe auch gewährleistet ist, daß kein Gas aus dem Körperhohlraum unbeabsichtigt entweichen kann.

Das erfindungsgemäße Trokarsystem hat auch den wesentlichen Vorteil, daß der Trokartubus auch bei großen auf die Trokartubushülsenspitze wirkenden axial gerichteten Kräften stauchungsfrei geführt ist. Beim Einstich in eine Gewebeschicht nimmt der Trokarstössel den Trokartubus mit, ohne daß sich der Trokartubus entgegen der Plazierungsvorschubrichtung verschieben könnte. Sind der Trokartubus und der Trokarstössel erfindungsgemäß aufeinander abgestimmt, so können auch Tubusschäfte plaziert werden, die bezüglich axial wirkender Kräfte keine ausreichende Eigensteifigkeit aufweisen.

Wird der Trokartubus aus einem elastischen rohrförmigen Material gefertigt, das geeignet ist, Radialkräfte im erforderlichen Umfang aufzunehmen, so kann auf einfache Weise die Gasdichtigkeit sowohl im Schaftbereich wie auch im Ventilbereich mit der oder den gummielastischen Dichtungsscheiben gewährleistet werden und der Gefahr einer unerwünschten Lumenverengung längs des Trokartubus ist sicher entgegengewirkt. Bei der Auswahl des Schaftmaterials kann darauf geachtet werden, daß der Trokartubus bei entferntem Trokarstössel ein Biegeverhalten aufweist, das auch stark gebogene Instrumente aufnehmen kann und während der Einführung gebogener Instrumente bei konstantem Lumen eine unzulässige Knickbildung verhindert.

Bei einer bevorzugten Ausgestaltung der Erfindung ist der Trokartubus als mit elastischem Material ummantelte Wendel ausgebildet, deren einzelne Wendelwindungen voneinander beabstandet sind und das elastische Material verschließt den Zwischenraum zwischen den einzelnen Wendelwindungen über die axiale Länge des Trokartubus gesehen.

Die Wendel kann als Drahtwendelfeder ausgebildet sein, die von einem elastischen Material mit guten Gleiteigenschaften ummantelt ist. Die Drahtwendel verhindert zuverlässig, daß das Lumen beim Umbiegen des Trokartubus nicht unzulässig verändert wird und so das passieren eines Instruments nicht erschwert. Am freien patientenseitigen Ende kann die Wendel als Ring ausgebildet sein, der wie die übrige Drahteinlage längs des gesammten Trokartubus mit einem gewebeverträglichen elastischen Material ummantelt ist. Die Drahteinlage kann einen beliebigen Aufbau aufweisen und muß nicht zwangsläufig als Wendelfeder ausgebildet sein.

In weiterer Ausgestaltung der Erfindung weist der Trokarstössel eine größere axiale Länge als der Trokartubus im Ruhezustand auf.

Dies hat den Vorteil, daß der Trokartubus vor der Plazierung gelängt werden kann. Ist der Trokarstössel im Querschnitt gesehen bezüglich seines Durchmessers kleiner als der Trokartubus, so kann sich der Trokartubus bei einer axialen Dehnung im Durchmesser verjüngen. Dies erleichtert das Einführen des Trokarstössels in eine Gewebeschicht, weil bei reduziertem Gesamtdurchmesser der Einstichwiderstand ebenfalls reduziert wird.

In einer anderen bevorzugten Ausführungsform der Erfindung ist der Ventilmechanismus in dem Ventilgehäuse gehalten, das mindestens eine elastische Dichtung aufweist, die den Trokarstössel im Trokartubus gasdicht umgreift und das Lumen bei entferntem Trokarstössel gasdicht verschließt.

Werden als Ventilmechanismus ein oder mehrere Dichtungsscheiben verwendet, so ist eine geringe axiale Bauhöhe im Ventilbereich möglich und der Dichtungsbereich weist eine zusätzliche Flexibilität auf. Stärker bis stark gebogene chirurgische Instrumente beliebiger Art lassen sich bei vorgegebenem Lumen durch den Trokartubus problemfrei hindurchschieben, weil der oder die Dichtungsscheiben das Lumen im Bereich des Ventilmechanismus nicht reduzieren bzw. blockieren. Unter Druck können Dichtungsscheiben nachgeben und sie können bei Bedarf ohne erhöhten Kostenaufwand auch schnell ausgewechselt werden.

In weiterer Ausgestaltung der Erfindung ist das Ventilgehäuse zerlegbar aufgebaut.

Der Trokartubus ist in dem Ventilgehäuse geführt gehalten. Der Bund liegt auf dem Gehäuseboden auf der Innenseite des Ventilgehäuses auf. An den Bund können sich Stützringe bzw. Dichtungsscheiben anschließen. Das Ventilgehäuse ist über einen Gehäusedeckel geschlossen, der die Dichtungsscheibe oder Dichtungsscheiben auf den Bund des Tubusschaftes presst. Über Klammern kann der Gehäusedeckel am Ventilgehäuse gehalten sein. Über das Lösen der Klammern ist das Ventilgehäuse leicht zerlegbar und auch reinigbar. Defekte Einzelteile können schnell ausgewechselt werden. Als Dichtungsmittel können im Ventilgehäuse geeignete Dichtungsscheiben oder Dichtungsringe Verwendung finden.

In weiterer vorteilhafter Ausgestaltung der Erfindung sind alle Einzelkomponenten des Trokarsystems aus reinigbaren und sterilisierbaren Materialien gefertigt.

Dies hat den Vorteil, daß das erfindungsgemäße Trokarsystem mehrfach eingesetzt werden kann. Die Dichtungsmittel können bei Bedarf ausgewechselt werden, damit die Gasdichtigkeit des Systems stets gewährleistet ist.

In weiterer Ausgestaltung der Erfindung weist der Trokartubus an der Außenumfangsoberfläche, bevorzugt im Bereich des vom Patienten wegweisenden Endes, Retentionsmittel, wie beispielsweise Laschen, Flügel, ringformige Elemente oder eine aufblasbare Manschette auf. Dies hat den Vorteil, daß der erfindungsgemäße Trokartubus beispielsweise in der Bauchdecke eines Patienten noch zusätzlich fixierbar ist. Wird der gesetzte Trokartubus unsachgemäß verrückt, so wirken die Retentionsmittel der Bewegung entgegen. Eine wesentliche Eigenfixierung des erfindungsgemäßen Trokartubus wird auch schon dadurch erreicht, wenn sich der gedehnt plazierte Trokartubus durch das Entfernen des Trokarstössels auf seine ursprüngliche Länge versucht zurückzubilden. Dabei vergrößert sich der Außendurchmesser des Trokartubus und die Außenkontur des Trokartubus dringt in das unmittelbar angrenzende Gewebe ein. Der Trokartubus wird über diese Maßnahme sicher in Position gehalten.

Bei dem erfindungsgemäßen Trokarsystem können sich flexible Trokartuben vom Einstechvorgang bis zur Endplazierung am Trokarstösselspitzenbereich sicher abstützen und eine unzulässige Stauchung des Trokartubusmaterials wird vermieden. Über die Dichtungsmittel (Scheiben und/oder Ringe) wird eine geringe Bauhöhe im Bereich des Ventilmechanismus erreicht. Dies erlaubt auch das Einführen von Instrumenten mit größeren Krümmungsradien. Der erfindungsgemäße Trokartubus wird während der Plazierung zentriert und nach dem Entfernen des Trokarstössels selbsttätig fixiert. Eine definierte Vorspannung des Trokartubus und die Befestigung am Trokarstössel lassen sich vor der Anwendung vorbereiten. Eine unzulässige Überdehnung des Trokartubus in axialer Richtung ist auszuschließen. Neben der notwendigen Flexibilität ist die Gasdichtigkeit längs des gesamten Trokartubus und auch im Bereich des Ventilmechanismus gegeben, wobei eine leichte Reinigbarkeit und Zerlegbarkeit des Gesamtsystems ebenfalls gewährleistet ist. Einzelteile, wie die Dichtungsscheiben, sind gut zugänglich und lassen sich leicht austauschen.

Erfindungsgemäß können nicht nur Dichtungsringe sondern auch Dichtungsscheiben im Ventilgehäuse den Trokarstössel gasdichtend umfassen. Dichtungsscheiben haben den Vorteil, daß sie beim Zurückziehen des Trokarstössels das Lumen selbsttätig ausreichend gasdicht verschließen können.

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen erläutert. Es zeigt:
- Fig. 1: ein erfindungsgemäßes Trokarsystem bestehend aus einem Trokartubus mit einem Ventilgehäuse und einem Trokarstössel;
- Fig. 2: einen Trokartubus mit geöffnetem Ventilgehäuse, das die in der Figur gezeigten Einzelteile aufnimmt.

Die Figuren der Zeichnung zeigen den erfindungsgemäßen Gegenstand teilweise schematisiert und die gezeigten Abmessungen sind nicht als bindend maßstäblich zu verstehen.

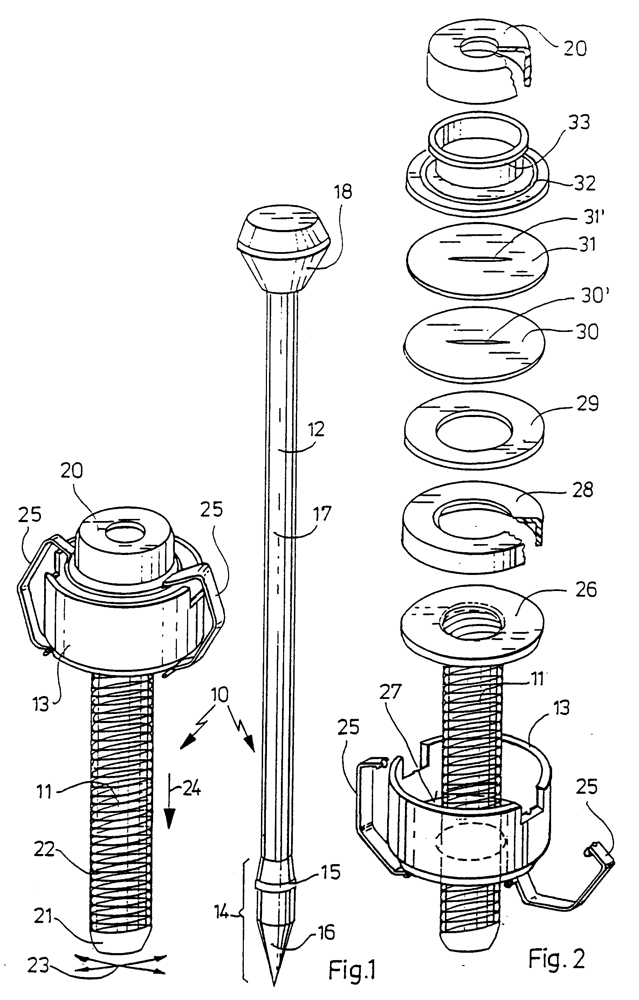

Fig. 1 zeigt mit 10 ein Trokarsystem, das sich aus einem Trokartubus 11, einem Trokarstössel 12 und einem Ventilgehäuse 13 zusammensetzt, das mit dem Trokartubus 11 verbunden ist. Im Spitzenbereich 14 des Trokarstössels 12 ist eine zirkuläre Schulter 15 ausgebildet, an die sich ein kegelförmiger Dorn 16 anschließt. Ein Schaftabschnitt 17 des Trokarstössels 12, der sich vom Spitzenbereich 14 bis zu einem Griff 18 erstreckt, weist einen geringeren Durchmesser als der Spitzenbereich 14 auf. Am Trokarstössel 12 ist dem Spitzenbereich 14 gegenüberliegend der Griff 18 ausgebildet, an dem der Trokarstössel 12 gefaßt und durch das Ventilgehäuse 13 hindurch in den Trokartubus 11 eingeschoben werden kann.

Am Ventilgehäuse 13 ist eine Dichtungskappe 20 ausgebildet, die aus einem gummielastischen Material gefertigt ist und den in das Ventilgehäuse 13 und in den Trokartubusschaft eingeführten Trokarstössel 12 dichtend umgreifen kann. Am patientenseitigen Ende 21 ist der Trokartubus 11 verjüngt ausgebildet.

Der Trokartubus 11 ist aus einer Wendel 22 gefertigt, die von einem gummielastischen Kunststoffmaterial ummantelt ist. Die einzelnen Wendelwindungen sind voneinander beabstandet und die Lücken zwischen den Wendelwindungen sind ebenfalls von dem Kunststoffmaterial ausgefüllt. Der Trokartubus 11 ist in Pfeilrichtungen 23 biegbar und er ist auch in Pfeilrichtung 24 dehnbar. Die Wendel 22 ist bevorzugt eine Metallwendel.

Wird ein Trokarstössel 12 in den Trokartubus 11 eingeführt, der eine größere Länge als der Trokartubus 11 mit dem Ventilgehäuse 13 aufweist, so kann der Trokarstössel 12 nur soweit in den Trokartubus 11 eingeführt werden, bis die zirkuläre Stufe oder Schulter 15 des Trokarstössels 12 formschlüssig in die Verjüngung am patientenseitigen Ende 21 des Trokartubus 11 eingreift. Wird der Trokarstössel 12 weiter in Pfeilrichtung 24 bei ortsfestem Ventilgehäuse 13 geschoben, so dehnt sich der Trokartubus 11 in Pfeilrichtung 24.

Das Ventilgehäuse 13 ist zerlegbar aufgebaut und wird über Klammern 25 zusammengehalten. In der Figur 1 sind zwei Klammern 25 gezeigt.

Fig. 2 zeigt einen Trokartubus 11 und ein Ventilgehäuse 13, das geöffnet und in die im Ventilgehäuse 13 angeordneten Einzelteile zerlegt ist. In das Ventilgehäuse 13 kann der Trokartubus 11 über die Verjüngung am patientenseitigen Ende 21 eingeschoben werden. Am Trokartubus 11 ist ein Bund 26 ausgebildet, der bei eingeschobenem Trokartubus 11 an einem Gehäuseboden 27 des Ventilgehäuses 13 anliegt. Ist der Trokartubus 11 in das Ventilgehäuse 13 eingefügt, so wird ein Stützring 28, aus einem zähelastischen bzw. formfesten Material, auf den Bund 26 zur Stabilisierung gelegt. Auf den Stützring 28 wird ein Dichtungsring 29 gelegt, auf dem sich die erste Dichtungsscheibe 30 und die zweite Dichtungsscheibe 32 abstützen. Die Dichtungsscheiben 30, 31 sind mit Schlitzen 30',31' versehen, damit bei geschlossenem Ventilgehäuse 13 der Trokarstössel 12 durch das Ventilgehäuse 13 hindurchgeführt werden kann. Die Schlitze 30',31' können übereinanderliegend um beispielsweise 90° verdreht zueinander angeordnet sein. Das Ventilgehäuse 13 ist über einen Gehäusedeckel 32 verschließbar, der über die Klammern 25 auf die Einbauteile im Ventilgehäuse 13 gedrückt ist. Am Gehäusedeckel 32 ist ein rohrförmiger Absatz 33 ausgebildet, auf den die gummielastische Dichtungskappe 20 aufsteckbar ist.

An der Außenoberfläche des Trokartubus 11, können Rückhaltemittel vorgesehen sein, die den Trokartubus 11 lagestabil im Gewebe fixieren. Als Rückhaltemittel eignen sich Laschen bzw. ringförmige Bünde, die sich beim Zurückziehen des Trokartubus 11 aus dem Gewebe öffnen und eine Gegenkraft bilden. Wird die Kraft beim Zurückziehen des Trokartubus 11 größer als die maximale Gegenkraft, so können die hier angesprochenen Rückhaltemittel in Ausziehrichtung des Trokartubus 11 umkippen und sich an die Außenoberfläche anlegen. Ist die maximale Sperrkraft der Rückhaltemittel überwunden, so kann der Trokartubus 11 ohne weiteren erhöhten Kraftaufwand aus dem Gewebe entfernt werden. Als Rückhaltemittel eignen sich auch auf der Außenfläche des Trokartubus angebrachte Wendeln oder eine aufblasbare Manschette. Die Rückhaltemittel sind bevorzugt unterhalb des Ventilgehäuses 13 und in einem gewissen Abstand davon über eine gewisse axiale Länge am Trokartubusschaft ausgebildet.

Ein Trokarsystem 10 setzt sich aus einem Trokartubus 11, einem Trokarstössel 12 und einem Ventilgehäuse 13 zusammen, das am vom Patienten wegweisenden Ende des Trokartubus 11 ausgebildet ist. Der Trokartubus 11 ist aus einem flexiblen Material hergestellt, das durch eine Wendel 22 stabilisiert ist. Der Trokartubus 11 weist am patientenseitigen Ende 21 eine Verjüngung auf, die sich an einer zirkulären Schulter 15 bei in den Trokartubus 11 eingeführtem Trokarstössel 12 abstützen kann. Der Trokartubus 11 ist in Pfeilrichtungen 23 biegbar und in Pfeilrichtung 24 dehnbar.

## Patentansprüche

1. Trokarsystem, das einen ein Lumen begrenzenden, biegbaren Trokartubus (11), einen Trokarstössel (12) und einen Ventilmechanismus (13) aufweist, der am vom Patienten wegweisenden Ende des Trokartubus (11) ausgebildet ist und durch dessen Ventilgehäuse (13) hindurch der Trokarstössel in den Trokartubus (11) einführbar ist, dadurch gekennzeichnet, daß der Trokartubus (11) bezüglich der axialen Länge dehnbar ist, daß der Trokartubus (11) am patientenseitigen Ende eine Verjüngung (21) aufweist, die eine zirkuläre Stufe oder einen konusförmig verlaufenden Abschnitt an der Spitze des Trokarstössels (12) formschlüssig umgreift.

2. Trokarsystem nach Anspruch 1, dadurch gekennzeichnet, daß der Trokartubus (11) als mit elastischem Material ummantelte Wendel (22) ausgebildet ist.

3. Trokarsystem nach Anspruch 2, dadurch gekennzeichnet, daß einzelne Wendelwindungen der Wendel (22) von einander beabstandet sind und daß das elastische Material den Zwischenraum zwischen den Wendelwindungen über die axiale Länge des Trokartubus (11) verschließt.

4. Trokarsystem nach einem der Ansprüche 1 bis 3, dadurch gekennzeichnet, daß der Trokarstössel (12) eine größere axiale Länge als der Trokartubus (11) im Ruhezustand aufweist.

5. Trokarsystem nach einem der Ansprüche 1 bis 4, dadurch gekennzeichnet, daß der Ventilmechanismus in dem Ventilgehäuse (13) gehalten ist, das mindestens eine elastische Dichtung (30, 31) aufweist, die den Trokarstössel (12) im Trokartubus (11) gasdicht umgreift und das Lumen bei entferntem Trokarstössel (12) gasdicht verschließt.

6. Trokarsystem nach einem der Ansprüche 1 bis 5, dadurch gekennzeichnet, daß das Ventilgehäuse (13) zerlegbar aufgebaut ist.

7. Trokarsystem nach einem der Ansprüche 1 bis 6, dadurch gekennzeichnet, daß alle Einzelkomponenten des Trokarsystems aus reinigbaren und sterilisierbaren Materialien gefertigt sind.

8. Trokarsystem nach einem der Ansprüche 1 bis 7, dadurch gekennzeichnet, daß der Trokartubus (11) an der Außenumfangsoberfläche, bevorzugt im Bereich des vom Patienten wegweisenden Endes, Retentionsmittel, wie Laschen, Flügel, ringförmige Elemente oder eine aufblasbare Manschette, ausweist.

## Claims

1. Trocar system having a flexible trocar tube (11) defining a lumen, a trocar plunger (12) and a valve mechanism (13) formed at the end of the trocar tube (11) facing away from the patient through the valve housing (13) of which the trocar plunger (12) can be guided into the trocar tube (11), characterized in that the trocar tube (11) can be stretched along its axial length, with the trocar tube (11) having a taper (21) at the end next to the patient which is in matched engagement with a circular step or a conical section at the tip of the trocar plunger (12).

2. Trocar system according to claim 1, characterized in that the trocar tube (11) is configured as a spiral (22) jacketed with elastic material.

3. Trocar system according to claim 2, characterized in that individual spiral windings of the spiral (22) are separated from another and the elastic material closes the intermediate space between the spiral windings along the axial length of the trocar tube (11).

4. Trocar system according to one of the claims 1 through 3, characterized in that the trocar plunger (12) has a larger axial length than the trocar tube (11) in the non-operative state.

5. Trocar system according to one of the claims 1 through 4, characterized in that the valve mechanism is held within the valve housing (13), with same having at least one elastic seal (30, 31) which surrounds the trocar plunger (12) within the trocar tube (11) in a gas-tight fashion and which closes the lumen in a gas-tight fashion when the trocar plunger (12) is removed.

6. Trocar system according to one of the claims 1 through 5, characterized in that the valve housing (13) can be disassembled.

7. Trocar system according to one of the claims 1 through 6, characterized in that all individual components of the trocar system are manufactured from materials which can be cleaned and sterilized.

8. Trocar system according to one of the claims 1 through 7, characterized in that the trocar tube (11) has retention means on the outer peripheral surface, preferentially in the vicinity of the end facing away from the patient, such as tabs, wings, ring-shaped elements or an inflatable cuff.

## Revendications

1. Système de trocart comportant un tube de trocart (12) flexible limitant un lumen, un poussoir de trocart (12) et un mécanisme de soupape (13) qui est réalisé à l'extrémité du tube de trocart (11) éloignée du patient et qui comporte un boîtier de soupape (13) à travers lequel le poussoir de trocart est susceptible d'être enfilé dans le tube de trocart (11), caractérisé en ce que le tube de trocart (11) est extensible quant à sa longueur axiale, en ce que le tube de trocart (11) présente un rétrécissement (21) à l'extrémité côté patient, qui entoure avec coopération de formes un gradin circulaire ou un tronçon s'étendant côniquement au niveau de la pointe du poussoir de trocart (12).

2. Système de trocart selon la revendication 1, caractérisé eu ce que le tube de trocart (11) est réalisé sous forme d'une hélice (22) enveloppée avec un matériau élastique.

3. Système de trocart selon la revendication 2, caractérisé en ce que les spires individuelles de l'hélice (22) sont écartées les unes des autres, et en ce que le matériau élastique referme l'intervalle entre les spires de l'hélice sur la longueur axiale du tube de trocart (11).

4. Système de trocart selon l'une quelconque des revendications 1 à 3, caractérisé en ce que, à l'état de repos, le poussoir de trocart (12) présente une longueur axiale supérieure à celle du tube dé trocart (11).

5. Système de trocart selon l'une quelconque des revendications 1 à 4, caractérisé en ce que le mécanisme de soupape est retenu dans le boîtier de soupape (13), lequel comporte au moins, un joint élastique (30, 31) qui entoure le poussoir de trocart (12) dans le tube de trocart (11) de manière étanche aux gaz et qui referme le lumen de façon étanche aux gaz lorsque le poussoir de trocart (12) est enlevé.

6. Système de trocart selon l'une quelconque des revendications 1 à 5, caractérisé en ce que le boîtier de soupape (13) est conçu de façon démontable.

7. Système de trocart selon l'une quelconque des revendications 1 à 6, caractérisé en ce que tous les composants individuels du système de trocart sont fabriqués en des matériaux susceptibles d'être nettoyés et stérilisés.

8. Système de trocart selon l'une quelconque des revendications 1 à 7, caractérisé en ce que le tube de trocart (11) comporte sur la surface périphérique extérieure, de préférence dans la région de l'extrémité éloignée du patient, des organes de retenue, tels que des pattes, des ailettes, des éléments annulaires ou une manchette gonflable.
